# FASCICULE DE BREVET EUROPEEN

(11) **EP 1 414 371 B1**
(45) Date de publication et mention de la délivrance du brevet: **07.07.2010**
(21) Numéro de dépôt: 02767579.2
(22) Date de dépôt: 17.07.2002
(51) Int. Cl.: A61F 2/02, A61F 2/08

(54) **MICROMUSCLE EN MILIEU BIOLOGIQUE**
MIKROMUSKEL IN EINEM BIOLOGISCHEN MEDIUM
MICRO-MUSCLE IN BIOLOGICAL MEDIUM

(30) Priorité: 17.07.2001 FR 0109526
(43) Date de publication de la demande: 06.05.2004
(73) Titulaire: UNIVERSITE JOSEPH FOURIER, F-38041 Grenoble Cédex (FR)
(72) Inventeur: CINQUIN, Philippe, F-38700 La Tronche (FR); CINQUIN, Olivier, F-38700 La Tronche (FR); FAVIER, Denis, F-38410 Saint Martin d'Uriage (FR); ORGEAS, Laurent, F-38140 Reaumont (FR); PECHER, Matthieu, F-38240 Meylan (FR); PUJOL, Sonia, F-38000 Grenoble (FR)
(74) Mandataire: de Beaumont, Michel
(86) Numéro de dépôt international: PCT/FR2002/002556
(87) Numéro de publication internationale: WO 2003/007844

(56) Documents cités:
- WO-A-00/27814
- WO-A-00/78222
- WO-A2-02/38197
- US-A- 4 157 085
- US-A- 5 100 933
- US-A- 5 250 167
- MICROMUSCLE AB: "NEWS AND PRODUCTS" INTERNET, [en ligne] XP002197173 Extrait de l'Internet: <URL:http://www.micromuscle.com/html/news. html> [extrait le 2002-10-31]

## Description

La présente invention concerne des dispositifs pouvant servir d'actionneur ou d'endoprothèse temporaire ou définitive à l'intérieur d'un milieu biologique tel que le corps humain ou un corps animal.

La présente invention trouve notamment des applications comme endoprothèse annulaire ou "stent" pouvant servir à compenser une sténose artérielle, ou comme moyen d'intervention pour aider à des opérations de couture, d'agrafage ou d'assemblage de vaisseaux sanguins, pour obturer un vaisseau, pour remplir une cavité, ou pour faire agir un élément, tel qu'une aiguille contre une paroi interne d'un milieu biologique tel que le corps humain ou animal.

On utilise classiquement, pour réaliser de telles opérations, des ballons gonflables ou des dispositifs à mémoire de forme qui présentent tous des inconvénients comme cela sera exposé ci-après.

Le document WO 00/78222 décrit un micromuscle et des dispositifs médicaux utilisant des micromuscles composés de polymères dont les changements de forme sont activés électriquement.

Le document WO 02/38197 décrit des implants capables de changer de forme par effet osmotique dès leur implantation dans le milieu biologique.

Plus particulièrement, la présente invention prévoit un micromuscle destiné à être immergé dans un milieu biologique, comprenant une enceinte déformable dont une portion au moins est constituée d'une membrane semi-perméable, cette enceinte contenant un soluté susceptible d'être osmotiquement actif, cette enceinte étant conçue pour avoir, après gonflage par effet osmotique, une forme prédéterminée, dans lequel le soluté est activable par un produit injectable dans le milieu biologique.

Selon un mode de réalisation de la présente invention, le soluté est lié à une matrice d'attachement dont il peut se détacher par suite d'une compétition avec un autre corps.

Selon un mode de réalisation de la présente invention, le soluté est accroché à des molécules d'HABA, elles-mêmes reliées à des matrices d'attachement par des protéines, telles que des dérivés d'avidine ou de streptavidine, cette liaison pouvant être rompue par compétition avec de la biotine ou analogue.

Selon un mode de réalisation de la présente invention, le soluté est lié à des molécules d'avidine, elles-mêmes reliées à des matrices d'attachement par des particules d'HABA, cette liaison pouvant être rompue par compétition avec de la biotine ou analogue.

Selon un mode de réalisation de la présente invention, les molécules de biotine ou analogue sont susceptibles d'être monomères ou dimères.

Selon un mode de réalisation de la présente invention, le soluté est encapsulé dans au moins une enveloppe susceptible d'être détruite par une réaction physique ou chimique.

Selon un mode de réalisation de la présente invention, le soluté est constitué de macromolécules susceptibles d'être brisées par action physique ou chimique.

Selon un mode de réalisation de la présente invention, ladite enceinte comprend une première partie en un matériau élastique de forme souhaitée, communiquant avec une deuxième partie constituant une membrane semi-perméable, par exemple sous forme de fibres.

Selon un mode de réalisation de la présente invention, ladite enceinte ou sa première partie élastique est en forme de tore.

Selon un mode de réalisation de la présente invention, ladite enceinte ou sa première partie élastique est susceptible de se déformer longitudinalement.

Selon un mode de réalisation de la présente invention, ladite enceinte ou sa première partie élastique est un volume déformable sensiblement sphérique.

Selon un mode de réalisation de la présente invention, ladite enceinte sous sa première partie élastique est constituée de deux demi-cylindres coulissant l'un à l'intérieur de l'autre.

Selon un mode de réalisation de la présente invention, ladite enceinte ou sa première partie élastique est entourée d'une gaine ou filet déterminant sa forme définitive.

Selon un mode de réalisation de la présente invention, le soluté est de l'albumine.

Selon un mode de réalisation de la présente invention, la membrane semi-perméable du micromuscle est en communication avec une deuxième enceinte constituée d'un matériau souple et contenant une réserve de liquide susceptible de former une solution avec un soluté contenu dans la première enceinte.

Selon un mode de réalisation de la présente invention, la deuxième enceinte contient un soluté susceptible d'être libéré par action physique ou chimique.

La présente invention prévoit aussi un dispositif d'insertion d'aiguilles dans une paroi d'un vaisseau rempli d'un liquide biologique, comprenant un guide, des aiguilles solidaires d'un fil d'un premier côté du guide, ces aiguilles pouvant être couchées sur le guide, des premiers micromuscles osmotiques du premier côté du guide, susceptibles de redresser les aiguilles, et au moins un second micromuscle osmotique disposé du côté opposé du guide.

La présente invention prévoit aussi un dispositif d'aide à l'agrafage comprenant un micromuscle insérable à l'extrémité d'un vaisseau sectionné, comprenant un corps cylindrique, des extrémités digitées.

La présente invention prévoit aussi une utilisation du micromuscle susmentionné, dans laquelle le micromuscle est sous forme torique et est destiné à servir de joint au niveau du raccord entre deux conduits, tels qu'une endoprothèse cylindrique et un vaisseau sanguin.

La présente invention prévoit aussi une utilisation du micromuscle susmentionné, dans laquelle le micromuscle est sous forme torique, dilatable vers l'intérieur et est destiné à obturer un conduit ou une endoprothèse cylindrique disposée dans ce conduit.

La présente invention prévoit aussi une utilisation du micromuscle susmentionné, dans laquelle le micromuscle est sous forme d'un sac ou cylindre dilatable vers l'extérieur et est destiné à obturer un conduit.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A à 1C illustrent un premier mode de réalisation d'un micromuscle selon la présente invention ;
les figures 2A à 2C illustrent un deuxième mode de réalisation d'un micromuscle selon la présente invention ;
les figures 3A à 3C illustrent des variantes d'un troisième mode de réalisation d'un micromuscle selon la présente invention ;
les figures 4A et 4B représentent un quatrième mode de réalisation d'un micromuscle selon la présente invention ;
la figure 5A représente un cinquième mode de réalisation d'un micromuscle selon la présente invention ;
la figure 5B représente un sixième mode de réalisation d'un micromuscle selon la présente invention ;
les figures 6A et 6B représentent un septième mode de réalisation de la présente invention ;
les figures 7 à 9 illustrent un exemple d'application de la présente invention à une opération d'anastomose termino-latérale ;
les figures 10 à 12 illustrent un exemple d'application de la présente invention à une opération d'agrafage ; et
les figures 13A et 13B illustrent un exemple d'application de la présente invention à une opération d'obturation d'un vaisseau.

La présente invention prévoit un micromuscle ou micromoteur pouvant fonctionner en milieu biologique. Ce micromuscle comprend une enceinte constituée au moins partiellement d'une membrane semi-perméable permettant le transfert d'une solution par osmose et contenant un soluté susceptible d'entrer en solution avec un fluide contenu dans le milieu biologique. Ce soluté sera choisi pour ne pas pouvoir traverser ladite membrane semi-perméable. Sa présence induit une pression osmotique susceptible d'entraîner des transferts de liquide vers l'enceinte ; le soluté sera donc qualifié d'"osmotiquement actif".

L'enceinte peut être placée sous forme comprimée ou repliée dans une zone choisie d'un milieu biologique, par exemple à un niveau donné d'une artère, par l'intermédiaire d'un cathéter ou d'un endoscope. Une fois le micromuscle mis en place, des molécules de la solution dans laquelle il est placé tendent à traverser la membrane semi-perméable et le micromuscle reprend une forme désirée sous l'effet de la pénétration d'un liquide biologique, par exemple de l'eau, à l'intérieur de l'enceinte.

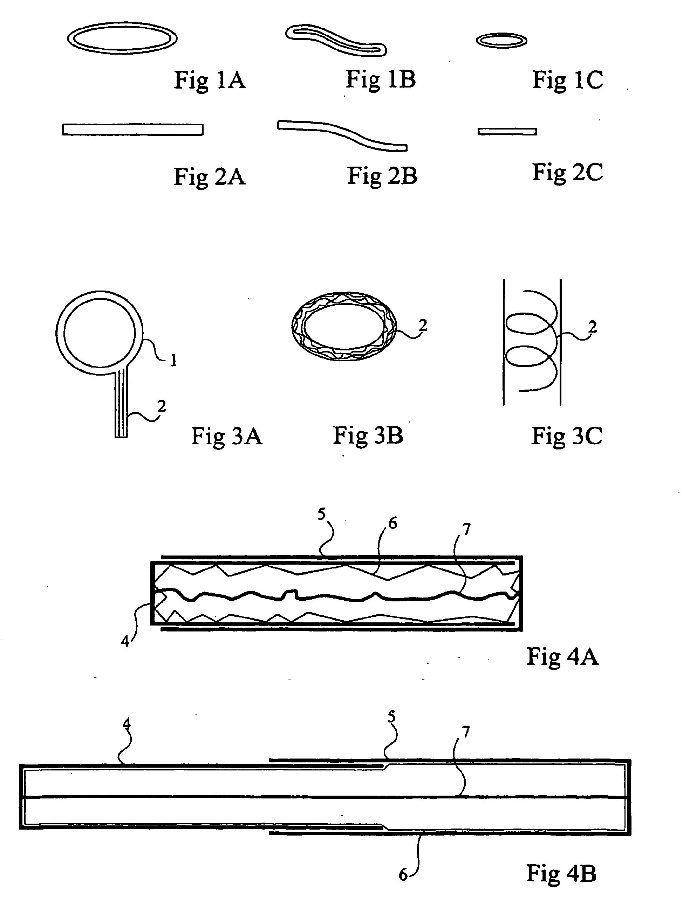

Des exemples de formes et de structures de micromuscles sont donnés en figures 1 à 6.
La figure 1A est une vue en perspective d'un micromuscle de forme torique en expansion. Au repos, ce micromuscle a ou bien une forme aplatie et éventuellement repliée telle que représentée en figure 1B ou bien une forme comprimée ou réduite comme cela est représenté en figure 1C.
La figure 2A est une vue de côté d'un micromuscle de forme cylindrique en expansion. Au repos, ce micromuscles a ou bien une forme aplatie et éventuellement repliée comme cela est représenté à la figure 2B ou bien une forme cylindrique de volume comprimé ou réduit comme cela est représenté en figure 2C.

L'enceinte telle qu'illustrée par exemple en figure 1A et 2A peut être constituée d'une membrane semi-perméable, éventuellement constituée d'une couche mince semi-perméable déposée sur un matériau support poreux ou perforé. Cette membrane peut être élastique ou non. Par exemple, pour passer de la forme représentée en figure 1B ou 2B à la forme représentée en figure 1A ou 2A, le matériau de l'enceinte n'a pas besoin d'être élastique. Par contre, dans le cas des figures 1C et 2C, le matériau de l'enceinte est un matériau élastique qui se dilate de façon adaptée.

Divers moyens peuvent être prévus pour contrôler la forme finale et/ou la dilatation de l'enceinte. Par exemple, l'enceinte sera munie de fibres ou entourée d'un filet pour contrôler sa forme finale après dilatation (passage du liquide du milieu biologique vers l'intérieur de l'enceinte).

Comme l'illustre la figure 3A, l'enceinte peut être composée sur une partie de sa surface d'une membrane semi-perméable non dilatable et peu ou pas déformable, le reste de sa surface étant constitué d'un matériau déformable et éventuellement élastique lié à la membrane semi-perméable. On pourra utiliser une enceinte 1, par exemple de forme torique, en un matériau élastique, dont le volume interne communique avec le volume interne de fibres 2 telles que les fibres couramment utilisées dans les opérations d'hémodialyse. Le liquide pénétrant dans les fibres 2 ira remplir l'enceinte 1 et la dilatera sous l'effet de la pression osmotique. Ce mode de réalisation mixte comportant une première partie constituée d'une membrane élastique, par exemple en le produit commercialisé sous la marque Silastic, et une seconde partie constituée d'une membrane semi-perméable, éventuellement en forme de fibres, de préférence souples, pourra s'adapter à la plus grande partie des modes de réalisation de la présente invention comme le notera l'homme de l'art.

Le micromuscle peut également être constitué de fibres fermées à parois semi-perméables, initialement dans un état replié, qui tendent à se redresser et à se raidir sous l'effet de la pression osmotique.

En figure 3B, ces fibres, 2, sont disposées à l'intérieur d'une enceinte torique déformable.

En figure 3C, ces fibres, 2, sont disposées directement à l'intérieur d'un élément cylindrique auquel on veut appliquer une force de dilatation, par exemple un vaisseau sanguin. Dans les deux cas, les fibres, contenant initialement des molécules incapables de traverser la membrane en concentration supérieure à la concentration du fluide biologique en molécules elles-mêmes incapables de traverser la membrane, tendront à se gonfler et à se redresser et donc à solliciter de façon centrifuge la surface qui les entoure, une fois placées dans un fluide biologique. Pour que les fibres prennent, une fois raidie la forme hélicoïdale illustrée en figure 3C, on pourra les fixer par endroit, éventuellement à coulissement, avant gonflage à une gaine souple non représentée ou aux parois du vaisseau.

Dans une première version, un ensemble de fibres osmotiques parallèles sont cousues au tissu qui forme la paroi. Le trajet d'une quelconque de ces fibres a une forme spiralée. Lorsque la pression augmente à l'intérieur de la fibre, cette dernière tendra à maximiser son volume, et aura donc tendance à diminuer son rayon de courbure. Les deux extrémités de la fibre osmotique sont solidement amarrées à un point particulier de la paroi. Par contre, la couture de la fibre osmotique à la paroi est suffisamment lâche pour que la fibre puisse glisser par rapport à la paroi. Le diamètre interne de l'hélice peut donc augmenter, et la paroi aura tendance à se dilater. La forme prise par l'ensemble résultera de l'équilibre des forces centripètes exercées par les fibres osmotiques et de la résistance à la dilatation exercée par la paroi.

Diverses variantes de cette version peuvent être envisagées. Par exemple, des fils inextensibles parallèles aux génératrices du cylindre que forme la paroi peuvent être fixés de diverses manières (couture, collage...) à cette dernière. La fixation est réalisée de sorte à ménager des espaces réguliers entre les fils inextensibles et la paroi, par lesquels les fibres peuvent passer. Ces fils inextensibles pourront éventuellement être des tiges semi-rigides.

Dans une deuxième version, les fibres osmotiques sont directement intégrées au tissu, à la trame de laquelle elles participent. Un ensemble de fibres, par exemple de Dacron, est disposé de manière à former une série de fils parallèles. Les fibres osmotiques sont ensuite croisées avec cette série de fils parallèles. Diverses formes de motifs pourront être créées par le croisement entre fibres de Dacron et fibres osmotiques (par exemple, les fibres osmotiques pourront avoir une forme hélicoïdale, une forme dessinant des chevrons...). Dans une variante, le tissu est constitué exclusivement de fibres osmotiques.

On notera qu'un tel dispositif peut trouver des applications en dehors du domaine de la conception d'endoprothèses vasculaires. En effet, un sac composé d'un tel tissu peut se comporter comme une pompe, dont les mouvements sont conditionnés par la concentration relative du milieu liquide de l'intérieur du sac et du milieu intérieur des fibres en substances osmotiquement actives.

Les figures 4A et 4B représentent un quatrième mode de réalisation de la présente invention, respectivement dans un état où le micromuscle est contracté, tel qu'il est au moment de sa mise en place, et dans un état où il est dilaté, après sa mise en place et établissement de la pression osmotique à l'intérieur de l'enceinte. Deux demi-cylindres 4 et 5 coulissent l'un à l'intérieur de l'autre. Chacun des demi-cylindres 4 et 5 est fermé à son extrémité opposée à l'autre demi-cylindre. A l'intérieur du demi-cylindre interne 4 sont disposées une ou plusieurs enceintes à paroi semi-perméable 6 à l'intérieur desquelles se trouvent des molécules incapables de traverser la membrane, non représentées. Les extrémités opposées des deux demi-cylindres sont liées par un fil 7 pour limiter leur extension. A titre d'exemple, le système à l'état replié peut avoir un diamètre de l'ordre de 100 µm et une longueur de l'ordre de 1 à 3 mm, cette longueur étant sensiblement double dans l'état déplié. Le dispositif des figures 4A et 4B peut être qualifié de micromoteur, un élément actif tel qu'une aiguille pouvant être lié à l'une de ses extrémités.

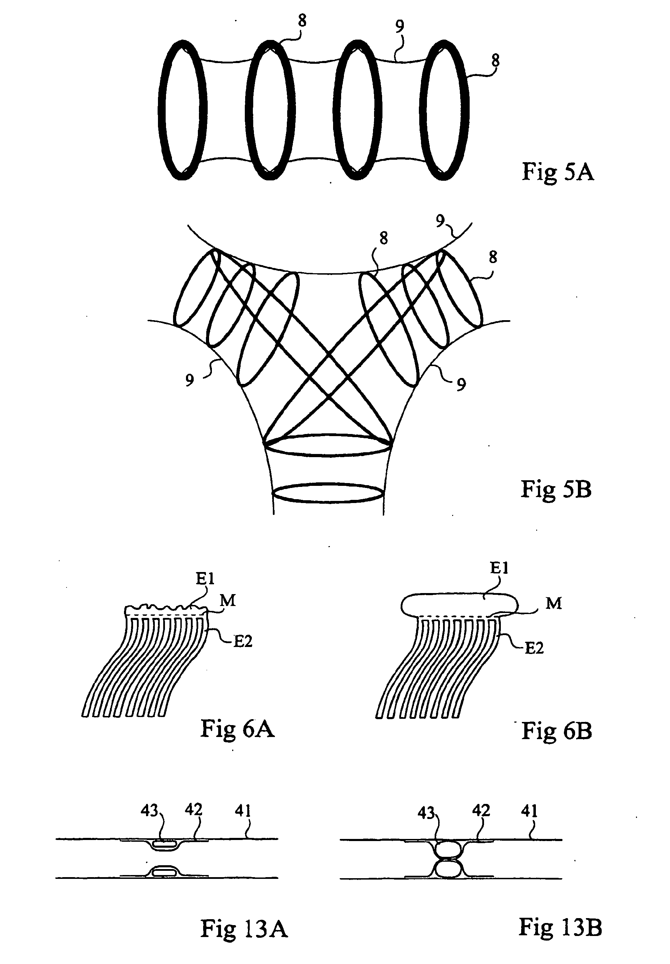

La figure 5A représente un autre mode de réalisation de la présente invention dans lequel plusieurs micromuscles osmotiques en forme de tore 8 selon la présente invention sont reliés les uns aux autres par un filet ou des fils souples 9, par exemple en Dacron. Les tores peuvent être éloignés les uns des autres comme cela est représenté ou être adjacents.

La figure 5B représente un autre exemple de micromuscle composite, les micromuscles toriques élémentaires étant désignés par la référence 8 et les fils ou filets de liaison par la référence 9.

L'homme de l'art notera que le micromuscle selon la présente invention est susceptible de nombreuses autres réalisations, notamment de nombreuses autres formes. Il pourra par exemple s'agir d'un sac susceptible de se dilater et d'épouser des parois internes d'une cavité dans lequel il est placé, par exemple pour remplir une cavité telle qu'un anévrisme d'un vaisseau cérébral ou autre.

Divers solutés, non nocifs pour l'organisme en cas de fuite, pourront être utilisés. Un soluté connu est par exemple l'albumine que ne laissent pas passer les membranes d'hémodialyse.

Les figures 6A et 6B illustrent un septième mode de réalisation de la présente invention utilisable en particulier quand le micromuscle selon la présente invention doit être inséré dans un milieu biologique éventuellement humide mais non liquide. Dans ce cas, il risque de ne plus être possible de "pomper" l'eau de l'organisme. Il est alors prévu un dispositif comprenant deux enceintes ou compartiments E1 et E2 communiquant par une membrane osmotique M. Dans un état initial, le compartiment E2 contient un fluide et le compartiment E1 contient un soluté susceptible d'entrer en solution avec le fluide du compartiment E2. Ainsi, comme l'illustre la figure 6B, le compartiment E1 aura tendance à se "gonfler" par pénétration à travers la membrane M du fluide contenu dans le compartiment E2, le soluté contenu dans le compartiment E1 venant en solution avec ce fluide. Chacun des compartiments a une enveloppe externe déformable, étanche et biocompatible. Dans l'exemple de réalisation illustré en figures 6A et 6B, l'enceinte E2 est solidaire d'un grand nombre de fibres étanches analogues à des cheveux ou tentacules incapables d'obstruer un conduit ou d'exercer une force sur ses parois. Par contre, le compartiment E1 n'aura pas d'effet sur les parois d'un conduit dans lequel il est disposé dans l'état "dégonflé" illustré en figure 6A mais aura un effet d'obturation ou de pression dans l'état "gonflé" représenté en figure 6B. Ce compartiment E1 peut avoir toute forme souhaitée, par exemple la forme d'un sac gonflable illustré dans les figures ou une forme torique, cylindrique, ou autre.

Pour insertion dans le corps humain, par exemple dans un conduit ou vaisseau que l'on veut obturer ou sur les parois duquel on veut exercer une action, le micromuscle des figures 6A et 6B sera d'abord placé dans un cathéter sous forme comprimée, c'est-à-dire que le compartiment E1 aura un volume réduit, le fluide qu'il contient étant chassé sous l'effet de la pression vers le compartiment E2. Dès que le dispositif est mis en place, le compartiment E1 est libre de se dilater et le fluide contenu dans le compartiment E2 vient remplir ce compartiment qui exerce alors une action souhaitée dans le milieu dans lequel il est placé.

On pourra prévoir diverses variantes de ce mode de réalisation. Par exemple, comme cela sera décrit ci-après, le compartiment E1 peut comprendre des molécules enfermées dans une enveloppe protectrice ou susceptible de réagir chimiquement avec d'autres molécules pour être activées seulement quand cela est souhaité. De même, le compartiment E2 peut contenir des molécules de soluté non libres et susceptibles d'être libérées seulement par suite d'une action physique ou chimique. Dans ce deuxième cas, il est possible de "dégonfler" le compartiment E1 après insertion et gonflage.

### CONTROLE DE LA DILATATION D'UN MICROMUSCLE OSMOTIQUE

Selon un aspect de la présente invention, il est prévu un procédé de contrôle de la dilatation d'un micromuscle selon la présente invention. Ce contrôle peut être chimique ou physique.

### Contrôle chimique

Selon un mode de réalisation de cet aspect de la présente invention, le micromuscle peut contenir un soluté A susceptible de réagir avec un corps B et de former avec celui-ci un soluté C, le nombre de molécules du soluté C formées étant supérieur au nombre de molécules du soluté A consommées lors de la réaction entre A et B. Le corps B est propre à traverser la paroi semi-perméable et les solutés A et C en sont incapables. On injecte alors dans le milieu biologique, par exemple au moyen d'une aiguille d'injection le corps B. On comprendra que, en procédant à des injections successives, on pourra procéder à des "gonflages" successifs du micromuscle. On pourra ainsi, par exemple si on souhaite dilater une artère, procéder d'abord à une dilatation légère puis augmenter la dilatation ultérieurement. L'intervalle entre deux injections peut être long et sera choisi en fonction des réactions de l'organisme au micromuscle.

Une mise en oeuvre de ce mode de réalisation se fonde sur la notion de "compétition" entre corps chimiques. Le principe est qu'un corps A peut se combiner, par exemple d'une manière non-covalente, avec deux corps B et C. Si on met en présence A et C dans un solvant, il va donc se former un complexe A-C. Si maintenant on introduit B dans la solution, et si B a une plus grande "affinité" pour A que celle de C pour A, B va "déplacer" C : les molécules C vont être libérées, et il se formera un complexe A-B.

Ce principe de compétition peut être utilisé pour augmenter la force osmotique, par exemple de la manière suivante. On utilise des dérivés de protéines particulières, telles que l'avidine ou la streptavidine, liées de manière covalente à des "matrices d'attachement" (corps solide quelconque servant de support). Ces protéines jouent le rôle du corps A pour lequel va se produire une compétition entre le corps B et le corps C. Ces protéines A présentent la particularité de pouvoir se lier d'une manière non-covalente avec une molécule de faible poids moléculaire, le HABA (acide 4-hydroxyazobenzène-2-carboxylique). Le corps C sera obtenu en "greffant" le HABA sur une molécule d'un poids moléculaire suffisant pour ne pas traverser la membrane semi-perméable. Au moment de son introduction dans l'organisme, l'enceinte contient donc une matrice d'attachement, à laquelle est lié d'une manière non covalente, par l'intermédiaire des protéines A, le corps C. C étant fixé à la matrice n'est donc pas en solution et n'a pas d'effet osmotique. On fait alors pénétrer dans l'organisme un dérivé de "biotine", qui joue le rôle du corps B. La biotine est une vitamine de faible poids moléculaire, naturellement présente dans l'organisme. La biotine présente la particularité d'avoir une affinité très forte pour l'avidine et pour la streptavidine, beaucoup plus forte que l'affinité du HABA pour ces protéines. Ainsi, des molécules du corps C se détachent de la matrice d'attachement et constituent un soluté qui tend à augmenter la pression de solution à l'intérieur de l'enceinte.

On choisira A, ainsi que B, de manière à obtenir une affinité sélective de B pour A : ces molécules auront une affinité réciproque très forte, alors que la biotine naturelle aura une affinité moins forte pour A. Le faible poids moléculaire de B lui permettra de traverser facilement la membrane semi-perméable. L'affinité de B pour A l'amènera à entrer en compétition avec C pour se fixer sur A, libérant ainsi de la matrice le HABA, et donc le corps C, qui pourra exercer son effet osmotique.

On notera que l'on peut fixer à la matrice non pas un dérivé de l'avidine ou de la streptavidine, mais un dérivé du HABA, à condition de prendre pour C le résultat d'une fusion d'une "grosse" molécule et d'un dérivé de l'avidine ou de la streptavidine. Comme dans le cas précédent, l'injection d'un analogue de biotine "libérera" C, par compétition avec le HABA.

Cette variante, dans laquelle C comporte un dérivé d'avidine ou de streptavidine, présente l'avantage de permettre une réversibilité partielle de l'effet osmotique désiré. On sait en effet produire des molécules comportant non pas un seul site ayant un effet analogue à celui de la biotine, mais deux tels sites. Soit B' une telle molécule (que l'on qualifiera de forme "dimère" d'analogue de biotine, par opposition à la forme "monomère" d'analogue de biotine précédemment utilisée, qui ne dispose que d'un site de fixation pour un dérivé de l'avidine ou la streptavidine). Après que C a été libéré de la matrice (par injection d'une forme monomère d'analogue de biotine), si l'on injecte une forme dimère d'analogue de biotine, la forme dimère va entrer en compétition avec la forme monomère. Si la concentration en forme dimère est suffisante, elle va déplacer les formes monomères des sites de dérivés d'avidine ou de streptavidine de C. Ceci va se traduire par la formation de complexes C-B'-C, réduisant ainsi le nombre de molécules en solution, et donc l'effet osmotiques. On peut répéter le processus, en injectant à nouveau en quantité suffisante la forme monomère d'analogue de biotine. Dans ce cas en effet, la compétition va devenir favorable à la formation de complexes C-B, ce qui augmente à nouveau le nombre de molécules en solution.

Bien que la méthode ait été décrite dans l'objectif de contrôler l'effet osmotique d'un micromuscle, elle s'applique également à la libération contrôlée de principes actifs, fournissant ainsi une alternative aux dispositifs de type "seringue implantable". De tels dispositifs existent, et sont constitués de "réservoirs" contenant le produit. Ce dernier est éjecté de ces réservoirs sous l'effet d'une source d'énergie physique (gaz sous pression, pression osmotique, rupture de la paroi du réservoir sous l'effet d'ultrasons...). Le principe de compétition précédemment décrit présente dans son application à ces dispositifs l'avantage de permettre un contrôle précis du moment où le principe actif est libéré (avantage par rapport aux seringues à gaz ou à effet osmotique), et de ne nécessiter l'utilisation d'aucun appareil capable de produire et de diriger la source d'énergie externe (avantage par rapport aux méthodes utilisant par exemple des ultrasons). Il suffit pour atteindre cet objectif particulier que le corps C qui sera libéré soit le principe actif dont on veut contrôler la libération, et qu'il puisse traverser l'enceinte dans laquelle il se trouve. Dès que le compétiteur B adéquat est introduit dans l'organisme (par exemple par voie sub-linguale, par ingestion ou par injection), il va libérer le corps C, lui permettant ainsi de diffuser dans l'organisme et d'y exprimer son effet médical.

On notera que, dans cette variante de l'invention, l'utilisation d'une matrice n'est plus indispensable, dans la mesure où l'on ne vise plus le contrôle de l'effet osmotique. Dans ce cas, soit C le résultat de la combinaison entre le principe actif et du HABA, et soit A le résultat de la fusion entre un dérivé de l'avidine et une "grosse" molécule. A et C, mis en présence dans la même solution, forment un complexe A-C. Le dispositif inséré dans l'organisme comporte une enceinte limitée par une membrane imperméable à A-C, mais perméable à C, ainsi qu'au dérivé B de la biotine. L'introduction de B dans l'organisme va déplacer C, qui pourra donc diffuser dans l'organisme.

On notera par ailleurs que l'enceinte utilisée n'est pas nécessairement artificielle. Il existe en effet dans l'organisme des régions dans lesquelles ne peuvent pénétrer que certaines classes très particulières de molécules. Les frontières naturelles de ces régions peuvent donc jouer le rôle de la membrane semi-perméable précédemment mise en oeuvre. Le liquide céphalo-rachidien est un exemple d'une telle région, qui présente l'avantage d'être facilement accessible (par exemple par ponction lombaire). On peut donc utiliser une telle région comme "réservoir", et y introduire, par exemple par ponction, un principe actif C tel que celui présenté au paragraphe précédent. On choisira dans ce cas le couple (C, B) de telle sorte que B soit susceptible d'atteindre la région d'intérêt. On notera que, dans le cas particulier du liquide céphalo-rachidien, la libération de C se fera principalement dans les zones de résorption du liquide céphalo-rachidien, qui sont situées dans le cerveau. Cela est particulièrement intéressant si l'activité préférentielle de C s'exprime dans le cerveau (L-DOPA, par exemple, précurseur de la dopamine utilisée dans le traitement de la maladie de Parkinson).

On notera également qu'une variante de l'exploitation des propriétés de compétition offertes par les dérivés de biotine peut être mise en oeuvre. Soit une protéine possédant une grande cavité interne pouvant servir de réservoir (c'est par exemple le cas de certaines protéines "chaperonnes" comme GroEL, ou de la ferritine). Il est possible de configurer cette protéine pour que des molécules d'HABA soient situées au niveau de son "ouverture", et pour que la fixation de protéines d'avidine ou de streptavidine soit susceptible de fermer la cavité. Un tel mécanisme permet "d'encapsuler" une grande quantité de molécules de principe actif. L'introduction dans l'organisme de biotine déplace une partie des "bouchons" d'avidine, libérant ainsi une partie des molécules encapsulées. Ce système nécessite que les complexes "protéine-réservoir" et "protéine-bouchon" soient dans un milieu qui assure leur stabilité et une protection du système immunitaire, et que ce milieu soit sensible aux variations sanguines du taux de biotine. Ces conditions peuvent être imposées dans une enceinte artificielle isolée de l'organisme par une membrane adéquate, ou obtenues dans une enceinte naturelle comme le liquide céphalo-rachidien.

On notera enfin que les exemples proposés s'appuient sur le cas particulier de la compétition entre dérivés de la biotine et dérivés d'avidine ou de streptavidine, mais que les mêmes résultats peuvent être obtenus avec d'autres systèmes de compétition entre molécules.

### Contrôle physique

Selon un mode de réalisation de cet aspect de la présente invention, le contrôle peut être effectué de façon physique, par exemple en plaçant une partie au moins du soluté dans une ou plusieurs microcapsules de sorte qu'il n'a initialement pas ou peu d'activité. On pourra ensuite briser, éventuellement sélectivement, les microcapsules au moyen d'une source d'énergie externe, par exemple des ultrasons, un champ magnétique ou un laser. On pourra également injecter un corps susceptible de dissoudre les microcapsules.

On notera qu'une énergie physique peut être directement utilisée pour "casser" des molécules en molécules plus petites, ce qui a un effet sur les propriétés osmotiques de la solution ainsi traitée. Des macromolécules comme l'ADN peuvent être cassées par l'application d'ultrasons (c'est une technique couramment utilisée en biologie moléculaire). Plus les ultrasons sont appliqués longtemps, plus la taille moyenne des fragments diminue. Dans une variante de la présente invention, on place des fragments d'ADN de grande taille (obtenus par exemple par un protocole quelconque de purification de l'ADN bactérien, ou par "réaction en chaîne polymérase" à partir du matériel génétique de l'individu à traiter) à l'intérieur d'une membrane semi-perméable choisie pour ne pas laisser passer ces fragments de grande taille. Après application d'ultrasons, le nombre de fragments augmente, et par voie de conséquence la force osmotique. La membrane peut être choisie de manière à laisser passer des fragments d'ADN de taille suffisamment réduite. Dans ce cas, une application suffisamment prolongée d'ultrasons permet de libérer une partie de l'ADN de l'intérieur de la membrane, et donc de diminuer la force osmotique. On notera toutefois que dans cette variante on ne pourra plus par la suite augmenter de nouveau la force osmotique.

### EXEMPLES D'APPLICATIONS

On va maintenant décrire divers exemples d'applications d'un micromuscle à gonflage osmotique selon la présente invention.

### 1. Endoprothèses vasculaires

On connaît des procédés pour traiter un anévrisme, c'est-à-dire une perte de parallélisme des bords d'un vaisseau, qui se traduit par une dilatation d'un segment vasculaire, consistant à insérer dans le vaisseau, à hauteur de la dilatation, un cylindre, par exemple en un matériau à mémoire de forme, qui se met en place de façon à obturer la communication entre le vaisseau et l'anévrisme. Toutefois, on s'aperçoit en pratique qu'au cours du temps, le vaisseau se dilate et le sang circulant dans le vaisseau peut à nouveau entrer en communication avec l'anévrisme.

Pour résoudre ce problème, la présente invention prévoit d'utiliser au lieu du cylindre à mémoire de forme, un cylindre formé à partir d'un micromuscle osmotique selon l'invention. Selon une variante de l'invention, on prévoit d'utiliser une endoprothèse vasculaire classique constituée d'un matériau à mémoire de forme, et de disposer aux deux extrémités de cette prothèse un micromuscle osmotique de forme torique qui jouera le rôle de joint d'étanchéité de diamètre variable et contrôlable. En effet, comme cela a été indiqué précédemment, on pourra prévoir de gonfler de manière progressive ce micromuscle torique, à intervalles réguliers, pour tenir compte des déformations du vaisseau.

### 2. Dilatation vasculaire progressive

Des méthodes vasculaires connues de dilatation de vaisseaux se basent sur l'utilisation de "stents" ou ressorts annulaires que l'on insère à l'intérieur d'un vaisseau sanguin à un emplacement où celui-ci est sténosé. Ces stents utilisent classiquement des alliages à mémoire de forme et se heurtent au problème de "re-sténose", à savoir que, après avoir été augmenté par le geste de dilatation, le diamètre du vaisseau diminue secondairement faisant ainsi perdre au patient le bénéfice de l'intervention.

La demanderesse considère que cette re-sténose est en grande partie liée au caractère agressif de la dilatation. En effet, par les procédés classiques, cette dilatation se fait très rapidement, dans certains cas en quelques secondes pour des stents à mémoire de forme et en quelques minutes pour des stents gonflés mécaniquement. Le diamètre du vaisseau peut être amené à augmenter d'un facteur de l'ordre de 2 ou plus. Il s'ensuit une réaction inflammatoire qui va exercer sur le stent un effort constrictif suffisant pour le déformer.

L'utilisation d'un micromuscle en forme de tore selon la présente invention permet de résoudre ce problème étant donné qu'il peut être réglé pour se "gonfler" très progressivement, par exemple en plusieurs jours. D'autre part, comme cela a été indiqué précédemment, on peut prévoir des procédés d'activations successives du micromuscle selon l'invention et, à des intervalles réguliers, par exemple d'une semaine, d'un mois ou plus, "regonfler" le micromuscle pour procéder à une dilatation très progressive du vaisseau à dilater.

Un micromuscle selon la présente invention peut se présenter sous un particulièrement petit volume à l'état non contracté et pourra donc facilement être inséré par des moyens classiques tels que des cathéters ou des endoscopes à tout emplacement souhaité.

### 3. Suture de vaisseaux

### 3.1. Anastomose termino-latérale

Le pontage coronarien utilise habituellement une dérivation d'une artère (par exemple l'artère mammaire interne), qui est mobilisée et anastomosée juste en aval de la zone sténosée (anastomose termino-latérale). Les diamètres des coronaires les plus fines sur lesquelles de tels gestes sont réalisés sont de l'ordre de 1,5 mm à 2 mm. La présente invention prévoit un dispositif permettant d'effectuer une couture tout en respectant la pression régnant dans le vaisseau (ici la coronaire), pour en faciliter la manipulation.

### 3.1.1 Préparation de la mammaire

L'artère mammaire peut être "équipée" de divers mécanismes destinés à faciliter son anastomose avec la coronaire. Il est en effet possible d'introduire un cathéter dans l'extrémité libre de la mammaire, et de gonfler un ballonnet autour de ce cathéter, ce qui permettra de fixer à cette extrémité les mécanismes adaptés à ceux dont sera ultérieurement équipée la coronaire (au prix éventuellement du sacrifice ultérieur des quelques millimètres de vaisseau situés en aval du ballonnet terminal).

### 3.1.2 Réalisation d'une "boutonnière" sur la coronaire

On introduit à l'intérieur de la coronaire, au niveau souhaité pour l'anastomose, un cathéter dont les dimensions sont inférieures au millimètre. Cette introduction peut se faire par voie fémorale.

Le cathéter comporte un guide. Ce guide pourra être immobilisé en deux points situés en amont et en aval de la zone à anastomoser (au moyen de deux pinces venant saisir la coronaire).

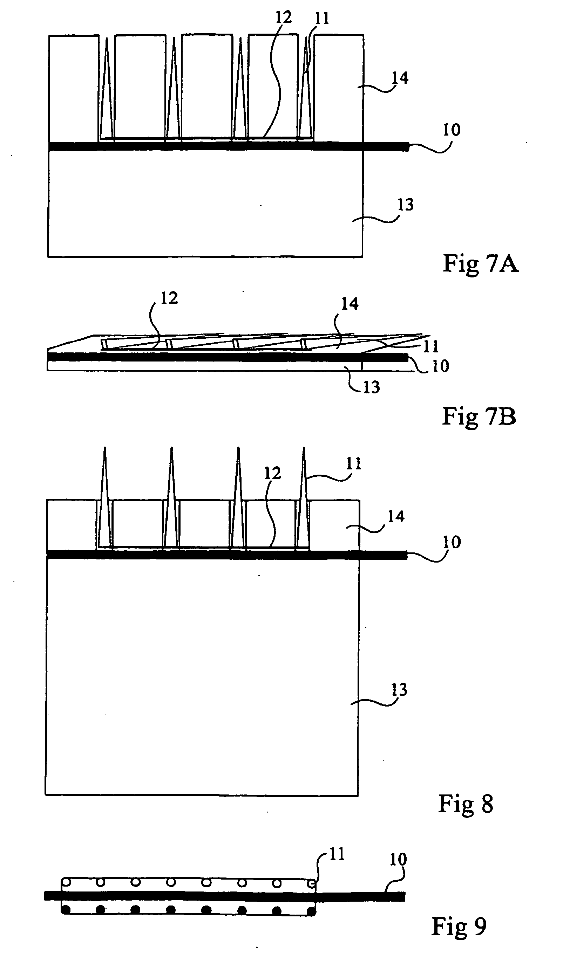

Sur ce guide 10, est montée une "herse repliable". Cette herse est illustrée en position dépliée en figure 7A. Elle est composée de deux rangs d'aiguilles 11. Les formes et dimensions de ces aiguilles sont celles couramment utilisées pour permettre la couture des coronaires. Typiquement, leur diamètre à la base est de l'ordre de quelques centaines de micromètres, et leur longueur de l'ordre de quelques millimètres. A leur base, ces aiguilles sont solidaires d'un fil unique 12. Ce fil définit deux sous-ensembles d'aiguilles "droites" et "gauches". Le fil relie d'abord l'ensemble des aiguilles "droites", dont les bases sont alignées (ou forment une demi-ellipse très allongée), puis l'ensemble des aiguilles "gauches" (voir figure 9).

Les aiguilles sont disposées de telle sorte que la herse puisse avoir deux configurations extrêmes. Dans la configuration "cathéter", illustrée en figure 7B, les aiguilles sont allongées dans l'axe du cathéter. Dans la configuration "couture", illustrée en figure 7A, les aiguilles sont perpendiculaires à cet axe. Selon l'invention, l'érection de la herse fait appel à au moins un "moteur osmotique" comprenant un premier ballonnet 13 disposé du côté inférieur de la herse et un ou plusieurs deuxièmes ballonnets 14 disposés du côté supérieur de la herse et agencés pour entourer les aiguilles. On peut remplacer l'un ou l'autre des moteurs osmotiques 13 et 14 par son équivalent hydraulique ou pneumatique.

Une fois que la herse est en place, pour permettre la perforation de la coronaire, le ballonnet 13 situé sous la herse est "gonflé", alors que les "ballonnets" 14 destinés à ériger les aiguilles gardent leur pression initiale. On arrive alors à la position illustrée en figure 8.

Si l'on utilise un mécanisme "osmotique", la réalisation de ces opérations se fait d'une manière très naturelle. Les ballonnets sont en effet dégonflés au moment de l'introduction du cathéter. Ce dernier peut être protégé par un capuchon, qui évite que ces ballonnets soient au contact d'un milieu hydrique. Le retrait de ce capuchon met les ballonnets au contact du milieu sanguin, ce qui active le mécanisme osmotique. Si par exemple on a mis les corps osmotiquement actifs en concentration deux fois plus faible dans les ballonnets 14 destinés à dresser les aiguilles que dans le ballonnet 13 destiné à plaquer ces aiguilles contre la paroi opposée, on verra tout d'abord les aiguilles se redresser, puis se retrouver plaquées contre la paroi, et finalement perforer cette dernière.

### 3.1.3 Anastomose de la mammaire et de la coronaire

Il faut utiliser les aiguilles de la herse, une fois qu'elles ont perforé la paroi de la coronaire.

La mammaire peut avoir été équipée d'un mécanisme permettant d'enserrer ces aiguilles (par exemple, triple collier, chaque aiguille passant devant le premier collier, derrière le deuxième, puis devant le troisième). Ces colliers sont alors tendus, ce qui rend solidaires les colliers et les aiguilles.

La mammaire peut avoir été équipée d'un mécanisme équivalent à celui de la coronaire. Il faut alors solidariser les aiguilles "coronariennes" avec les aiguilles "mammaires". Divers mécanismes simples sont envisageables (soudure, collage, "entortillement" d'une aiguille sur l'autre, etc.).

On peut également envisager que les aiguilles "droites" soient toutes soulevées, de manière à former une série de "ponts" de fil, sous lequel on introduit un câble, dont les deux extrémités seront solidarisées avec la mammaire. Les mouvements à réaliser sont ici très simples. Lorsque les aiguilles "droites" ont été solidarisées de la mammaire, on réalise la même opération avec les aiguilles "gauches".

### 3.1.4 Ouverture de la communication entre mammaire et coronaire

Jusqu'à ce stade, la circulation dans la coronaire n'a pas été interrompue, et le flux artériel de la mammaire n'est pas encore relié à celui de la coronaire. Pourtant, la couture est maintenant terminée. Il reste à perforer la paroi de la coronaire, sur sa composante située entre les deux rangées d'aiguilles. La pression de la circulation "arrondira" cette ouverture.

Cette perforation peut se faire mécaniquement, à partir d'un cathéter intra-mammaire. Elle peut également s'envisager électriquement. Le guide de cathéter intra-coronaire se trouve en effet plaqué contre la zone à perforer. S'il est conducteur du courant, et dénudé sur cette zone (le reste étant isolé), un courant peut passer par le cathéter intra-mammaire et le cathéter intra-coronarien, l'ensemble jouant le rôle d'un bistouri électrique.

On dégonfle finalement les ballonnets, et on peut alors retirer les cathéters coronariens et mammaires.

### 3.2 Agrafage osmotique

En chirurgie vasculaire, l'agrafage est une alternative intéressante à la couture, car il permet "d'affronter" deux vaisseaux sans laisser de matériel au contact du lit vasculaire. Une agrafeuse mécanique a été décrite par T. Richard dans "AAA : Laparoscopic and/or endovascular repair?", Angio-Techniques 2001. L'extrémité du vaisseau est positionnée sur une "enclume", ce qui permet de retourner le bord libre, puis de disposer d'un support suffisamment rigide pour permettre l'appui des agrafes.

Les inconvénients de ce système sont :
- une relative complexité de l'agrafeuse,
- la nécessité d'utiliser un substitut vasculaire intermédiaire (en effet, l'agrafeuse doit pouvoir "entrer" par l'extrémité libre d'un vaisseau ; on "équipe" donc les deux parties terminales à anastomoser, puis un dispositif adéquat permet de rétablir la continuité),

### - la difficulté à miniaturiser le système.

Pour remédier à ces inconvénients, la présente invention propose un dispositif d'agrafage osmotique.

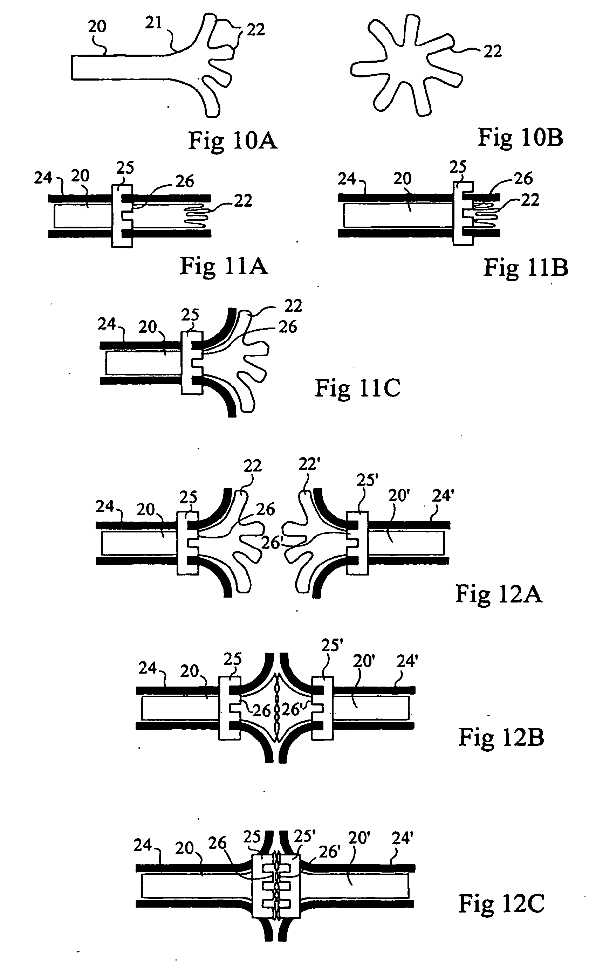

Dans un premier temps, on prépare l'extrémité libre de chaque vaisseau de la manière suivante. On introduit à l'intérieur du vaisseau un "sac" élastique, présentant sur une partie au moins de sa surface une membrane semi-perméable. Ce sac, lorsqu'il est gonflé, a la forme illustrée en figures 10A et 10B respectivement en vue de côté et en vue de face :
- il a une symétrie de révolution,
- une de ses extrémités 20 est cylindrique,
- le milieu 21 est conique,
- l'autre extrémité comprend des digitations 22.

Comme l'illustrent les figures 11A à 11C, ce sac est introduit "dégonflé" dans un vaisseau 24 (figure 11A). On installe sur le vaisseau 24 une bague 25 comportant autant de protubérances 26 que d'intervalles entre les digitations (figure 11B). Enfin le sac est "gonflé".

Les différentes étapes de l'assemblage de deux sections d'un vaisseau sont illustrées en figures 12A à 12C. Ces deux sections, équipées comme celle représentée en figure 11C, sont présentées en vis à vis comme l'illustre la figure 12A.

On a pris soin de positionner les protubérances 26 des bagues 25 dans les espaces laissés vides par les digitations 22. Les protubérances portées par une des bagues sont les parties "mâles" de "boutons pressions", celles portées par l'autre bague les parties "femelles" des mêmes "boutons pressions".

Les protubérances sont positionnées face à face, de façon complémentaire puis les bagues sont rapprochées (figure 12B) avant de solidariser les "boutons pressions" (figure 12C). Avant de bloquer le ou les derniers éléments de bouton pression, on dégonfle les sacs (par exemple en les perforant à l'aide d'une aiguille). Sous leur forme dégonflée, ils peuvent être extraits par l'orifice restant entre les deux vaisseaux.

Dans la présente section 3.2, on a considéré seulement le cas où les "sacs" destinés à dilater les orifices des vaisseaux et à préparer l'assemblage des bagues munies de protubérances étaient du type micromuscle osmotique. On notera que l'on pourrait également réaliser pour réaliser cette opération des ballons à gonflage pneumatique ou des dispositifs à mémoire de forme.

### 4. Obturation réversible d'un vaisseau

Un micromuscle selon la présente invention pourra également être utilisé à l'intérieur d'un conduit ou vaisseau comme valve que l'on peut ouvrir ou fermer.

Un mode de réalisation d'une telle valve est illustré en figure 13A en position ouverte et en figure 13B en position fermée à l'intérieur d'un conduit ou vaisseau 41. Une endoprothèse ou stent de forme cylindrique 42 réalisé par exemple en alliage à mémoire de forme, est entouré sur sa partie médiane d'un manchon 43 composé d'un micromuscle osmotique en forme d'anneau disposé entre le cylindre et le vaisseau. Dans le cas où les parois du vaisseau sont élastiques, le diamètre du stent est choisi légèrement supérieur à celui de ce vaisseau pour permettre un ancrage du stent dans le vaisseau. La capacité de dilatation du micromuscle, lors du contact avec la paroi du vaisseau, l'amène à exercer une force radiale qui comprime tout à la fois le stent et la paroi. On choisira les forces respectives exercées par le stent et par le micromuscle pour que ceci conduise à l'obturation complète du vaisseau. On pourra utiliser l'un des moyens précédemment décrits pour arrêter l'effet de compression exercé par le micromuscle et "rouvrir" le vaisseau.

Une telle valve pourra par exemple être utilisée à l'obturation réversible des trompes de Fallope. En effet, la ligature des trompes de Fallope (conduit naturel de quelques millimètres de diamètre qui relie les ovaires à l'utérus) est utilisée comme moyen contraceptif. Cependant, cette technique conduit à une stérilisation quasiment définitive, d'éventuelles interventions chirurgicales destinées à rétablir la fonctionnalité de ces trompes connaissant un taux d'échec élevé.

Si une valve du type susmentionné est insérée dans chacune des deux trompes, le dispositif fonctionne comme un moyen contraceptif. L'introduction du stent dans la trompe peut se faire par voie basse, ou éventuellement par coelioscopie. Lorsque la femme désire interrompre cet effet, la fonctionnalité de la trompe est simplement rétablie.

On peut craindre que le milieu interne aux trompes de Fallope bien qu'humide soit insuffisamment liquide pour avoir une action directe sur un micromuscle osmotique susceptible d'attirer un liquide à partir du milieu environnant. Dans ce cas, on choisira d'utiliser un micromuscles selon le septième mode de réalisation de la présente invention, illustré en relation avec les figures 6A et 6B, qui comprend sa propre réserve de liquide. Dans cette application, on utilisera de préférence un micromuscle réversible, c'est-à-dire dans lequel le deuxième compartiment est susceptible par suite d'une action physique ou chimique de "dégonfler" le premier compartiment. L'action de contraception est alors rendue réversible.

L'homme de l'art notera qu'il est également possible d'utiliser directement la capacité de jouer un rôle de "bouchon" du septième mode de réalisation de la présente invention. Dans ce cas, le micromuscle est inséré dans la trompe de Fallope, qu'il obture. Si plus tard la patiente désire à nouveau pouvoir avoir des enfants, un des modes de contrôle du micromuscle précédemment décrits est appliqué, et permet de dégonfler le compartiment E1. Plus rien ne "bloque" alors le micromuscle dans la trompe de Fallope, et il tombe naturellement dans la cavité utérine, où il sera évacué lors de la période menstruelle suivante. Ce mode de réalisation particulier présente l'inconvénient de nécessiter une nouvelle mise en place du dispositif, si ultérieurement la fonction contraceptive est à nouveau souhaitée. Il présente cependant l'avantage de ne pas laisser en place un corps étranger dans l'organisme, ce qui peut poser des problèmes psychologiques et physiologiques (la mobilité de la paroi tubaire est en effet un élément important de la fertilité féminine, et la présence permanente d'un stent pourrait l'affecter négativement).

Parmi les nombreuse variantes et modifications de la présente invention qui apparaîtront à l'homme de l'art, on notera que la plupart des applications des micromuscles osmotiques qui ont été décrites pourront être mises en oeuvre en utilisant des dispositifs ayant, dans ces applications, des fonctions analogues à celles de ces micromuscles osmotiques, par exemple des dispositifs pneumatiques ou à mémoire de forme.

## Revendications

1. Micromuscle destiné à être immergé dans un milieu biologique, comprenant une enceinte (1) déformable dont une portion au moins est constituée d'une membrane semi-perméable, cette enceinte contenant un soluté susceptible d'être osmotiquement actif, cette enceinte étant conçue pour avoir, après gonflage par effet osmotique, une forme prédéterminée, dans lequel le soluté est activable par un produit injectable dans le milieu biologique.

2. Micromuscle selon la revendication 1, **caractérisé en ce que** le soluté est lié à une matrice d'attachement dont il peut se détacher par suite d'une compétition avec un autre corps.

3. Micromuscle selon la revendication 2, **caractérisé en ce que** le soluté est accroché à des molécules d'HABA, elles-mêmes reliées à des matrices d'attachement par des protéines, telles que des dérivés d'avidine ou de streptavidine, cette liaison pouvant être rompue par compétition avec de la biotine ou analogue.

4. Micromuscle selon la revendication 2, **caractérisé en ce que** le soluté est lié à des molécules d'avidine, elles-mêmes reliées à des matrices d'attachement par des particules d'HABA, cette liaison pouvant être rompue par compétition avec de la biotine ou analogue.

5. Micromuscle selon la revendication 4, **caractérisé en ce que** les molécules de biotine ou analogue sont susceptibles d'être monomères ou dimères.

6. Micromuscle selon la revendication 1, **caractérisé en ce que** le soluté est encapsulé dans au moins une enveloppe susceptible d'être détruite par une réaction physique ou chimique.

7. Micromuscle selon la revendication 1, **caractérisé en ce que** le soluté est constitué de macromolécules susceptibles d'être brisées par action physique ou chimique.

8. Micromuscle selon la revendication 1, **caractérisé en ce que** ladite enceinte comprend une première partie en un matériau élastique de forme souhaitée, communiquant avec une deuxième partie constituant une membrane semi-perméable, par exemple sous forme de fibres (2).

9. Micromuscle selon la revendication 1, **caractérisé en ce que** ladite enceinte ou sa première partie élastique est en forme de tore.

10. Micromuscle selon la revendication 1, **caractérisé en ce que** ladite enceinte ou sa première partie élastique est susceptible de se déformer longitudinalement.

11. Micromuscle selon la revendication 1, **caractérisé en ce que** ladite enceinte ou sa première partie élastique est un volume déformable sensiblement sphérique.

12. Micromuscle selon la revendication 1, **caractérisé en ce que** ladite enceinte est constituée d'une fibre qui se raidit après gonflage.

13. Micromuscle selon la revendication 1, **caractérisé en ce que** ladite enceinte sous sa première partie élastique est constituée de deux demi-cylindres (4,5) coulissant l'un à l'intérieur de l'autre.

14. Micromuscle selon la revendication 1, **caractérisé en ce que** ladite enceinte ou sa première partie élastique est entourée d'une gaine ou filet déterminant sa forme définitive.

15. Micromuscle selon la revendication 1, **caractérisé en ce que** le soluté est de l'albumine.

16. Micromuscle selon la revendication 1, **caractérisé en ce que** la membrane semi-perméable (M) du micromuscle est en communication avec une deuxième enceinte (E2) constituée d'un matériau souple et contenant une réserve de liquide susceptible de former une solution avec un soluté contenu dans la première enceinte (E1).

17. Micromuscle selon la revendication 16, **caractérisé en ce que** la deuxième enceinte contient un soluté susceptible d'être libéré par action physique ou chimique.

18. Dispositif d'insertion d'aiguilles dans une paroi d'un vaisseau rempli d'un liquide biologique, **caractérisé en ce qu'**il comprend :
un guide (10),
des aiguilles (11) solidaires d'un fil (12) d'un premier côté du guide, ces aiguilles pouvant être couchées sur le guide,
des premiers micromuscles osmotiques (14) selon la revendication 1 du premier côté du guide, susceptibles de redresser les aiguilles, et
au moins un second micromuscle osmotique (13) selon la revendication 1 disposé du côté opposé du guide.

19. Dispositif d'aide à l'agrafage comprenant un micromuscle selon la revendication 18 insérable à l'extrémité d'un vaisseau sectionné, comprenant :
un corps cylindrique (20), et
des extrémités digitées (22).

## Claims

1. A micromuscle for immersion in a biological medium, comprising a deformable chamber (1) having at least a portion formed of a semipermeable membrane, this chamber containing a solute likely to be osmotically active, the chamber being designed to have, after inflating by osmotic effect, a predetermined shape, wherein the solute is activable by a product injectable into the biological medium.

2. The micromuscle of claim 1, wherein the solute is bound to an attachment matrix from which it can detach in consequence of a competition with another body.

3. The micromuscle of claim 2, wherein the solute is bound to HABA molecules, themselves bound to attachment matrixes by proteins, such as avidin or streptavidin derivatives, this bond being breakable by competition with biotin or the like.

4. The micromuscle of claim 2, wherein the solute is bound to avidin molecules, themselves bound to attachment matrixes by HABA particles, this bond being breakable by competition with biotin or the like.

5. The micromuscle of claim 4, wherein the molecules of biotin or the like are monomers or dimers.

6. The micromuscle of claim 1, wherein the solute is encapsulated in at least one envelope to be destroyed by a physical or chemical reaction.

7. The micromuscle of claim 1, wherein the solute is formed of macromolecules likely to be broken by physical or chemical action.

8. The micromuscle of claim 1, wherein said chamber comprises a first portion made of a resilient material of desired shape, communicating with a second portion forming a semipermeable membrane, for example in the form of fibers (2).

9. The micromuscle of claim 1, wherein said chamber or its first resilient portion is torus-shaped.

10. The micromuscle of claim 1, wherein said chamber or its first resilient portion is capable of being longitudinally deformed.

11. The micromuscle of claim 1, wherein said chamber or its first resilient portion is a substantially spherical deformable volume.

12. The micromuscle of claim 1, wherein said chamber is formed of a fiber that stiffens after inflating.

13. The micromuscle of claim 1, wherein said chamber under its first resilient portion is formed of two half-cylinders (4, 5) sliding one inside of the other.

14. The micromuscle of claim 1, wherein said chamber or its first resilient portion is surrounded with sheath or net determining its definitive shape.

15. The micromuscle of claim 1, wherein the solute is albumin.

16. The micromuscle of claim 1, wherein the semipermeable membrane (M) of the micromuscle is in communication with a second chamber (E2) formed of a flexible material and containing a reserve of a liquid likely to form a solution with a solute contained in the first chamber (E1).

17. The micromuscle of claim 16, wherein the second chamber contains a solute likely to be released by physical or chemical action.

18. A device for inserting needles into a wall of a vessel filled with a biological liquid, comprising:
a guide (10),
needles (11) to which a wire (12) is firmly attached on a first side of the guide, the needles being likely to be laid flat on the guide,
first osmotic micromuscles (14) of claim 1 on the first side of the guide, likely to erect the needles, and
at least one second osmotic micromuscle (13) of claim 1 arranged on the opposite side of the guide.

19. A device for aiding the clipping comprising a micromuscle of claim 18 insertable at the end of a cut up vessel, comprising:
a cylindrical body (20), and
digitized ends (22).

## Patentansprüche

1. Mikromuskel zum Eintauchen in ein biologisches Medium, der Folgendes aufweist: eine verformbare Kammer (1) mit wenigstens einem Teil, der aus einer semipermeablen Membran gebildet ist, wobei diese Kammer eine Lösung enthält, die mit Wahrscheinlichkeit osmotisch aktiv ist, wobei die Kammer so aufgebaut ist, das sie nach einem Aufblasen durch einen osmotischen Effekt eine vorbestimmte Form einnimmt, wobei die Lösung aktivierbar ist durch ein Produkt, das in das biologische Medium injizierbar ist.

2. Mikromuskel nach Anspruch 1, wobei die Lösung an eine Befestigungsmatrix gebunden ist, von der sie sich lösen kann als Konsequenz eines Wettkampfs mit einem weiteren Körper.

3. Mikromuskel nach Anspruch 2, wobei die Lösung an HABA-Moleküle gebunden ist, die wiederum selbst an Befestigungsmatrizen durch Proteine gebunden sind, wie beispielsweise Avidin oder Streptavidin Derivate, wobei diese Bindung lösbar ist durch einen Wettkampf mit Biotin oder ähnlichem.

4. Mikromuskel nach Anspruch 2, wobei die Lösung an Avidin Molekülen gebunden ist, die wiederum selbst an Befestigungsmatrizen durch HABA-Partikel gebunden sind, wobei diese Bindung lösbar ist durch einen Wettkampf mit Biotin oder ähnlichem.

5. Mikromuskel nach Anspruch 4, wobei die Moleküle aus Biotin oder ähnlichem Monomere oder Dimere sind.

6. Mikromuskel nach Anspruch 1, wobei die Lösung in einem Einschluss eingekapselt ist, der durch eine physikalische oder chemische Reaktion zerstörbar ist.

7. Mikromuskel nach Anspruch 1, wobei die Lösung aus Makromolekülen oder ähnlichem gebildet wird, die mit Wahrscheinlichkeit durch physikalische oder chemische Wirkung zerbrechbar sind.

8. Mikromuskel nach Anspruch 1, wobei die Kammer einen ersten Teil aufweist, der aus einem elastischen Material mit gewünschter Form besteht, und mit einem zweiten Teil kommuniziert, der eine semipermeable Membran z.B. in der Form von Fasern (2) bildet.

9. Mikromuskel nach Anspruch 1, wobei die Kammer oder ihr erster elastischer Teil ringförmig ist.

10. Mikromuskel nach Anspruch 1, wobei die Kammer oder ihr erster elastischer Teil in der Lage ist, längs verformt zu werden.

11. Mikromuskel nach Anspruch 1, wobei die Kammer oder ihr erster elastischer Teil im Wesentlichen ein sphärisch verformbares Volumen ist.

12. Mikromuskel nach Anspruch 1, wobei die Kammer aus einer Faser ausgebildet ist, die sich nach einem Aufblasen versteift.

13. Mikromuskel nach Anspruch 1, wobei die Kammer unter ihrem ersten elastischen Teil aus zwei Halbzylindern (4, 5) ausgebildet ist, die ineinander gleiten.

14. Mikromuskel nach Anspruch 1, wobei die Kammer oder ihr erster elastischer Teil mit einer Höhle oder einem Netz umgeben ist, die bzw. das seine schlussendliche Form bestimmt.

15. Mikromuskel nach Anspruch 1, wobei die Lösung Albumin ist.

16. Mikromuskel nach Anspruch 1, wobei die semipermeable Membran (M) des Mikromuskels in Verbindung mit einer zweiten Kammer (E2) steht, die aus einem flexiblen Material gebildet ist und eine Reserve einer Flüssigkeit enthält, die mit Wahrscheinlichkeit eine Lösung mit einer Lösung bildet, die in der ersten Kammer (E1) enthalten ist.

17. Mikromuskel nach Anspruch 16, wobei die zweite Kammer eine Lösung enthält, die mit Wahrscheinlichkeit durch physikalische oder chemische Wirkung freigesetzt wird.

18. Vorrichtung zum Einführen von Nadeln in eine Wand eines Gefäßes, das mit einer biologischen Flüssigkeit gefüllt ist, wobei die Vorrichtung Folgendes aufweist:
eine Führung (10),
Nadeln (11), an denen ein Draht (12) fest befestigt ist, und zwar in einer ersten Seite der Führung, wobei die Nadeln mit Wahrscheinlichkeit flach an der Führung anliegen,
erste osmotische Mikromuskeln (14) nach Anspruch 1 an der ersten Seite der Führung, die mit Wahrscheinlichkeit die Nadeln aufrichten, und wenigstens ein zweiter osmotischer Mikromuskel (13) nach Anspruch 1, der auf der entgegengesetzten Seite der Führung angeordnet ist.

19. Vorrichtung zum Unterstützen des Klippens, die einen Mikromuskel nach Anspruch 18 aufweist, der an dem Ende eines aufgeschnittenen Gefäßes einführbar ist, wobei die Vorrichtung Folgendes aufweist:
einen zylindrischen Körper (20), und
digitalisierte Enden (22).
